# EUROPEAN PATENT APPLICATION

(11) **EP 1 905 354 A1**
(43) Date of publication of application: **02.04.2008**
(21) Application number: 07253798.8
(22) Date of filing: 26.09.2007
(51) Int. Cl.: A61B 5/0444, A61B 5/0488, A61B 5/00, A61B 5/03, A61B 5/024, A61B 5/0402, A61B 5/04

(54) **Signal replication medical apparatus**

(30) Priority: 28.09.2006 US 529650
(71) Applicant: Tyco Healthcare Group LP, Mansfield, MA 02048 (US)
(72) Inventor: Meyer, Peter, Shrewsbury, Massachusetts 01545 (US); Zaiken's, Eliot, Sparta, New Jersey 07871 (US)
(74) Representative: Pratt, David Martin

(57) **Abstract**

The signal replication medical apparatus includes an input adapted to receive at least one medical signal transmitted via a first medical device coupled to the body of a patient, signal processing circuitry adapted to generate at least one replicated signal from the at least one medical signal, and an output. The at least one replicated signal is indicative of being transmitted via a second medical device different than the first medical device. The output is adapted to deliver the at least one replicated signal to an external device configured to receive signals from the second medical device.

## Description

### BACKGROUND

### Technical Field

The present disclosure relates to a medical apparatus. In particular, the present disclosure relates to a medical apparatus and method adapted for converting medical signals for use in maternal and fetal monitoring.

### Description of Related Art

Medical devices are widely used for measuring a wide variety of monitoring signals. Monitoring signals, or raw signals received from the patient by the medical device, typically contain specific bioinformation or data of interest to a clinician. Common medical signals include electrocardiogram (ECG) signals or fetal and maternal signals, such as for example, uterine temperature, intrauterine pressure, electric fetal electrocardiogram signals, etc. Such signals from the patient are gathered by various sensors and transmitted via electrical cables to a monitor. The monitor displays or records the selected bioinformation contained in the monitoring signal.

Unfortunately, some of the various sensors employed (e.g., electrode, electrode arrays, temperature sensors, pressure transducers, catheters, etc.) to gather the bioinformation are often incompatible with the monitor available to the clinician. For example, the monitor may be compatible with a single medical device, or family of medical devices, or configured to receive signals with specific electrical characteristics (i.e. 4-20 mA, 0-5 VDC, 0-200 mV, etc...). Other causes of incompatibility include inconsistencies in the hardware used with the various monitors and/or development of new sensor or medical equipment not adapted for use with existing monitors.

The aforedescribed incompatibility between the various sensors and an existing monitor, regardless of the reason, causes several problems. Clinicians may be required to stock several different monitors and/or sensors for use with a specific monitor, therefore increasing the cost of medical care. Physical replacement of a monitor sensor may increase the duration of time of the medical procedure. Finally, the cost of introducing newly developed sensors may be prohibitive in that such sensors may require the purchase of new monitors compatible with the newly developed sensors. Therefore, there exists a need for an apparatus that substantially eliminates or minimizes incompatibilities between a medical sensor and a monitor.

### SUMMARY

In accordance with the present disclosure, an apparatus for replicating a medical signal includes an input adapted to receive at least one medical signal transmitted via a first medical device coupled to the body of a patient, signal processing circuitry adapted to generate at least one replicated signal from the at least one medical signal, and an output. The at least one replicated signal is indicative of being transmitted via a second medical device different than the first medical device. The output is adapted to deliver the at least one replicated signal to an external device configured to receive signals from the second medical device.

The signal processing circuitry may include an analog to digital converter, a signal processor and a digital to analog converter. The analog to digital converter is adapted to convert the at least one medical signal to a digital format. The signal processor processes the at least one medical signal in a digital format wherein data is extracted from the at least one medical signal. The signal generator receives the data extracted by the signal processor and generates therefrom the at least one replicated signal. The data relayed by the at least one replicated signal replicates data potentially outputted by the second medical device. The digital to analog converter converts the at least one replicated signal to an analog format. Alternatively, the signal processing circuitry comprises an ASIC. The signal processor is adapted to select one signal of the at least one converted output signal for extracting and processing data relayed by the selected signal.

The first medical device may be selected from a group consisting of at least one medical electrode and a medical electrode sensor array. The second medical device may be a tocodynamometer, an intrauterine pressure catheter or an ultrasound transducer. In the alternative, the first and second medical devices may be selected from a group consisting of at least one medical electrode, a medical electrode sensor array, an abdominal strain gage, a tocodynamometer, an intrauterine pressure catheter, and an ultrasound transducer, a vacuum pressure sensor, a fetal pulse oximeter, a pH sensor, a cervical dilation sensor, a cervical effacement sensor, a cervical length sensor and a fetal station sensor.

One or more indicators for indicating functionality of the first medical device may be provided. The one or more indicators may be selected from a group consisting of a visual indicator, an auditory indicator and a tactile indicator. The one or more indicators preferably indicates the quality of the at least one medical signal transmitted via the first medical device.

An electrical cable for relaying the at least one signal to the signal processing circuitry is included. An electrical receptacle is adapted for mating with an end of the electrical cable for performing a diagnostic check on the cable.

The at least one output signal includes data selected from a group consisting of heart, fetal, and uterine activity data. The first medical device may be a fetal monitoring electrode and the at least one signal may be a fetal monitoring signal outputted by the fetal monitoring electrode. The at least one output signal includes a uterine activity signal, and further comprising means for shorting the uterine activity signal to zero.

Other embodiments are also envisioned.

### BRIEF DESCRIPTION OF THE DRAWINGS

Various embodiments of the present disclosure are described herein with reference to the drawings wherein:

**FIG. 1** is a view of a maternal and fetal monitoring system incorporating a signal replication medical apparatus in accordance with the present disclosure;

**FIG. 2** is an electrical schematic illustrating the components of the signal replication medical apparatus; and

**FIG. 3** is a programming flowchart illustrating functionality of the signal replication medical apparatus.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

In the discussion which follows, the term "cable" may incorporate a single conductor or may comprise an assembly of conductors arranged in any mode of operation known in the art. Connector refers to a single plug, receptacle, or other device capable of connecting to a cable, device or apparatus. A connector assembly refers to the connection between two connectors wherein the connectors facilitate connectivity between two cables, devices or apparatus, or any combination thereof. Connection or coupling between the two components may be mechanical, electro-mechanical or solely electrical without any mechanical means of connection. Such electrical coupling or connection may be infrared or incorporate electromagnetic wave principles. Thus, the term "connection" or "electrical connection" is to be construed as any electrical, mechanical connection or combination thereof known in the art.

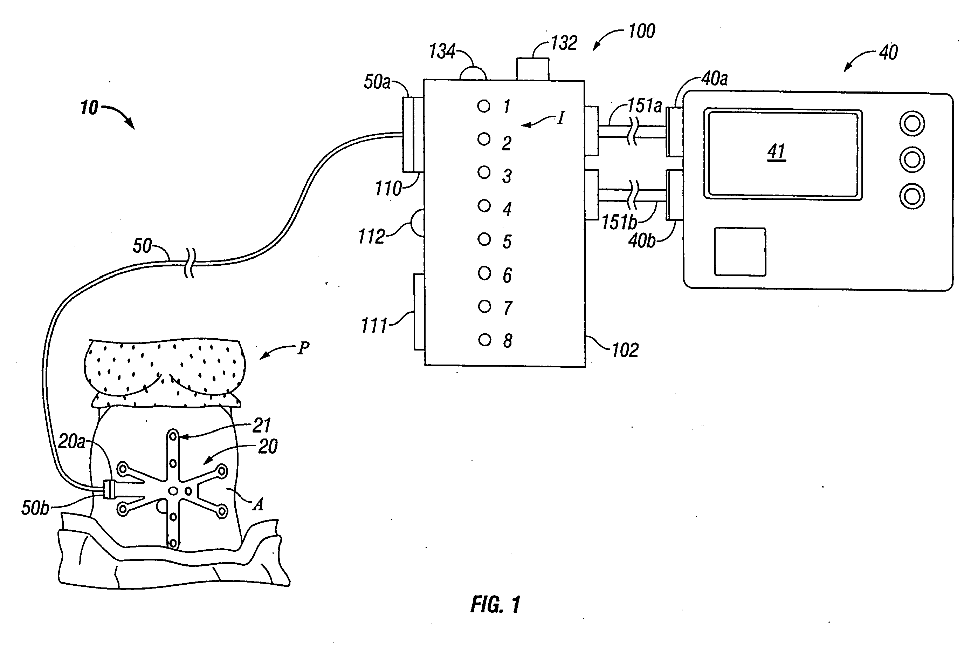

**FIG. 1** illustrates a maternal and fetal monitoring system **10** in conjunction with the signal replication medical apparatus **100** according to the present disclosure. The maternal and fetal monitoring system **10** generally includes an electrode array **20** and a monitor **40.** The electrode array **20** may include a plurality of electrodes **21** adapted to adhere to skin on the abdomen **A** of the patient **P.** One suitable electrode array in described in a U.S Provisional Application titled **Radial Electrode Array,** application number 60/798,842 and converted to a U.S. Utility Application, Attorney Docket Number H-KN-00380, (1502-124), concurrently filed on September 29, 2006 with the present application, the entire contents of which are incorporated herein by reference. The electrode array disclosed in this application includes a flexible substrate adapted to generally conform to a topography of a skin surface and having a central portion arranged about a central focal point and a plurality of finger-like projections extending radially outwardly from said central portion, a medical electrode disposed on at least one of the finger-like projections and a connector in electrical communication with the medical electrode and adapted to connect to an electronic system.

Typically, monitoring device **40** is configured to receive a monitoring signal from a specific type of medical device with the monitoring signals having specific electrical characteristics. For example, first input connector **40a** of monitoring device **40** is configured to receive a monitoring signal from an intrauterine pressure (IUP) catheter wherein the monitoring signal contains maternal intrauterine pressure medical signals. The electrical characteristics of a monitoring signal from an IUP catheter is typically an alternating current or direct current signal, as driven by the excitation voltage of the monitor to which it is connected, of amplitude less than 1 Volt. The amplitude of the IUP monitoring signal changes in response to changes in intrauterine pressure. Second input connector **40b** of monitoring device **40** may be configured to receive a monitoring signal from a fetal scalp electrode wherein the monitoring signal contains a fetal electrocardiogram (FECG) medical signal. The electrical characteristics of a monitoring signal from a fetal scalp electrode is typically an electrical potential of less than 1 Volt which characterizes the periodic polarization and depolarization of the fetal heart muscle.

In the maternal and fetal monitoring system **10** of **FIG. 1,** several incompatibilities exist between monitoring device **40** and the monitoring signals from electrode array **20.** Monitoring device **40** is physically incompatible with electrode array **20** since monitoring device **40** receives only two monitoring signals while electrode array **20** supplies eight monitoring signals. Monitoring device **40** is electrically incompatible with electrode array **20** since the signal characteristics of electrode array **20** are different than the signal characteristic required for the first and second input connectors **40a, 40b.** Finally, monitoring device **40** is configured to receive two monitoring signals, with each containing a single predominant medical signal, while electrode array **20** supplies eight monitoring signal, with each monitoring single containing a portion of several medical signals. With an electrode array **20,** the uterine electromyogram (EHG) and FECG medical signals must be extracted from the eight monitoring signals as described in U.S. Application 10/857,107 and U.S. Application 11/140,057, both to Marossero et al., the contents of which are incorporated herein by reference. Even if monitoring device **40** could physically and electrically receive monitoring signals from an electrode array **20,** monitoring device **40** lacks the processing capability to extract medical signals from the plurality of monitoring signals.

Signal replication medical apparatus **100,** as described herein, is used to resolve an incompatibility between any medical device and any monitoring device. Signal replication medical apparatus **100** consists of a housing **102** which houses a plurality of connectors **110, 111,** output cables **151A, 151B,** indicators **I** and user interface devices **112, 132, 134** described hereinbelow. Housing **102** may be sufficiently small and manufactured from lightweight materials, such as plastic, such that the signal replication medical apparatus **100** is a light-weight inline device.

Input connector **110** of the signal replication medical apparatus **100** is adapted to connect to a first end **50A** of an electrical cable **50.** Second end **508** of electrical cable **50** connects to a medical device, such as an electrode array **20.** It is envisioned signal replication medical apparatus **100** described herein may have any number of inputs and may connect to any number of medical devices.

First and second output cables **151A, 151B** of the signal replication medical apparatus **100** are adapted to connect to an electrical system, such as a monitoring device **40,** capable of receiving a monitoring signal. It is envisioned signal replication medical apparatus **100** described herein may have any number of outputs, in the form of output cables or output connectors, and may connect to any number of electrical systems capable of receiving a monitoring signal from a medical device.

Electrodes **21** on electrode array **20** receive monitoring signals from the patient and fetus, including signals from maternal uterine muscle and from the maternal and fetal heart muscles. Monitoring signals from electrodes **21** on electrode array **20** are transmitted through electrical cable **50** to the signal replication medical apparatus **100.** In this particular embodiment, electrical cable **50** transmits eight individual monitoring signals, one from each electrode **21** on electrode array **20,** to signal replication medical apparatus **100** with each monitoring signal containing at least a portion of several maternal and fetal medical signals.

First and second output cables **151a, 151b** of signal replication medical apparatus **100** connect to the first and second input connectors **40a, 40b,** respectively, of monitoring device **40.** Output signals from the signal replication medical apparatus **100** are transmitted on first and second output cables **51a, 51b** to monitoring device **40.**

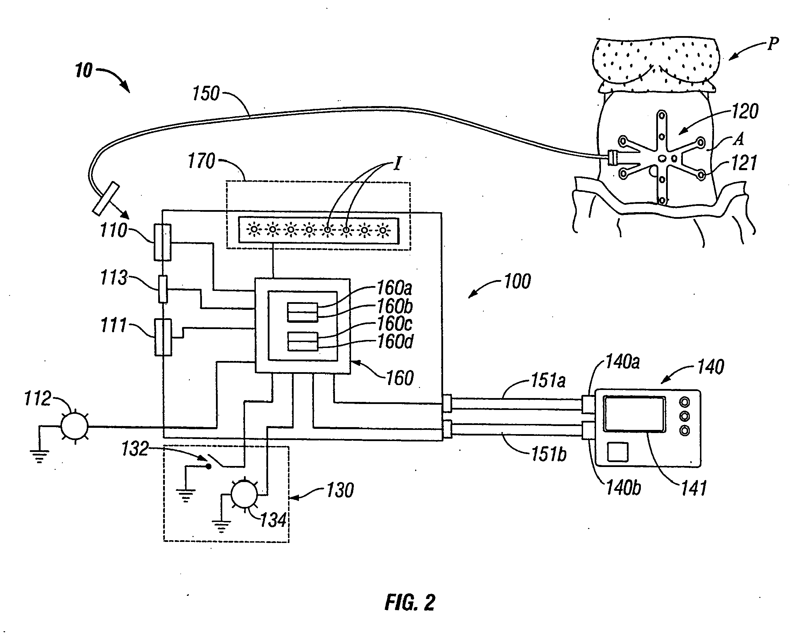

**FIG. 2** is an electrical schematic illustrating the components of the signal replication medical apparatus **100** in a maternal and fetal monitoring system **10** including an electrode array **120** positioned on the abdomen **A** of patient **P** and a monitoring device **140.** Signal replication medical apparatus **100** includes signal processing circuitry **160,** operably. coupled to the various input connectors, output cables, test connectors and indicator devices described hereinbelow.

Signal processing circuitry **160** may include an analog to digital (A/D) converter **160A,** a digital signal processor (DSP) **160B,** a signal generator **160C** and a digital to analog (D/A) converter **160D.** Signal processing circuitry **160** is adapted to receive at least one monitoring signal from a first medical device, such as an electrode array **120.** A/D converter 160A converts the analog monitoring signal from a first medical device to a digital representation of the analog monitoring signal. DSP **160B,** having a memory storing a set of programmable instructions capable of being executed by the DSP **160B** for performing the functions described herein, processes the converted monitoring signal. Processing may include the extraction of a medical signal from a monitoring signal, extraction of one or more medical signals from a plurality of monitoring signals or determination if the signal is a valid signal. Signal generator 160C receives at least a portion of processed data from DSP **160B** and generates replicated data indicative of a monitoring signal outputted by a second medical device. The replicated data is different than the monitoring signal from the first medical device and is compatible with monitoring device **140.** D/A converter **160D** converts the replicated data generated by the signal generator **160C** to an analog signal and analog signal is outputted to an output cable **151A, 151B.** Output cable **151A, 151B** transmits replicated analog signal to monitoring device **140;** and the medical signal is presented on display **141.**

Signal processing circuitry **160** may be an application-specific integrated circuit (ASIC) customized for this particular use or may be a general purpose device adapted for this use. The functions performed by the various elements **160A-D** of signal processing circuitry **160** may be performed in a variety of ways as known in the art

More specifically, first input connector **140A** on monitoring device **140** is configured to receive an IUP catheter monitoring signal containing a maternal intrauterine pressure medical signal. Second input connector **140B** is configured to receive a fetal scalp electrode monitoring signal, containing a FECG medical signal. Electrode Array **120** supplies eight monitoring signals to signal processing circuitry **160** with each monitoring signal containing at least a portion of several fetal and maternal medical signals (e.g. FECG medical signal, maternal ECG medical signal and maternal EHG medical signal). Electrode array **120** is therefore incompatible with monitoring device **140.**

Signal replication medical apparatus **100** receives the plurality of monitoring signals from electrodes **121** on electrode array **120** through electrical cable **150.** The A/D converter **160A** converts the monitoring signals, DSP **160B** extracts the EHG medical signal and the FECG medical signal from the monitoring signals. Signal generator **160C** replicated an EHG monitoring signal and an FECG monitoring signal indicative of a monitoring signal from an IUP catheter and a fetal scalp electrode, respectively. A/D converter **160D** converts the replicated IUP and FECG monitoring signals such that signals are compatible with first and second input connector **140A, 140B** of monitoring device **140.** The replicated monitoring signals are outputted through first and second output cable **151A, 151B,** received by first and second input connector **140A 140B,** respectively, of monitoring device **140** and the medical signals are presented on display **141.**

In another embodiment of the present disclosure, the medical signal may be altered by signal processing circuitry **160.** Alteration of the replicated signal may remove an incompatibility that exists between the signal and monitoring device **140,** may increase compatibility of the signal with the monitoring device **140** or may aid a clinician in recognizing a characteristic of the signal. For example, a FECG from an electrode array **120** may need to be inverted in order for the signal to be indicative of a signal received from a second medical device and compatible with monitoring device **140.** Alternatively, an offset may be added to the signal for the signal to be in a specific range (e.g. current or voltage range), or in order for a trigger or counting mechanism in the monitoring device **140** to recognize the signal. Low strength signals, or a portion of a low strength signal, may be amplified, re-scaled or otherwise altered. Alterations may be required to satisfy various criteria set by monitoring device **140** such as signal strength, signal quality, peak amplitude or signal energy.

In another embodiment of the present invention, signal replication medical apparatus **100** may replicate a signal indicative of a fault condition recognized by monitoring device **140.** For example, monitoring device **140** may indicate a fault condition on display **141** when the input is either open or shorted. Signal replication medical apparatus **100** may simulate this fault condition by replicating a signal indicative of an open or shorted medical device when signal processing circuitry **160** is unable to extract a medical signal from the monitoring signals. Signal replication medical apparatus **100** may replicate any such fault signal recognized by monitoring device **140.**

In another embodiment of the present disclosure, signal processing circuitry **160** performs at least one diagnostic check on electrical cable **150** as described in a U.S Utility Patent Application titled **Cable Monitoring Apparatus,** Attorney Docket Number H-KN-00513 (1502-143), concurrently filed on September 29, 2006 with the present application, the entire contents of which are incorporated herein by reference. Referring to **FIGS. 1** and **2,** signal processing circuitry **160** connects to various indicators **112** that indicate the results of a diagnostic check of electrical cable **150** attached between first input connector **110** and first diagnostic connectors **111.** Diagnostic check may include testing continuity and impedance of the various conductors and connectors, testing continuity and impedance between various conductors, testing capacitive properties of the cable or conductors, testing insulation in the cable, measuring losses in the cable and conductors, measuring frequency response and signal losses at various frequencies and any other test known in the art. Various indicators **112** are indicative of at least one operating feature of the electrical cable **150** which include test performed, or measurements made, on the cable. Indicators **112** may be audible indicators, visual indicators, or any indicators known in the art, or combination thereof.

First connector **110** may interface with various medical devices including a medical electrode, a medical electrode array, an abdominal strain gage, a tocodynamometer, an intrauterine pressure catheter, an ultrasound transducer, a vacuum pressure sensor, a pulse oximeter, a pH sensor, a cervical dilation sensor, a cervical effacement sensor, a cervical length sensor and a fetal station sensor. Signal replication medical apparatus **100** may receive monitoring signals from any number of medical devices and supply replicated monitoring signals to any number of monitoring devices.

In yet another embodiment of the present disclosure first connector **110** and second connector **111** may receive medical signal from a first medical device **120** and second medical device (not shown). Signal processing circuitry **160** may select the source of the replicated signal from the first input connector **110** or from the second input connector **111.** Selection may be performed automatically by the signal processing circuitry **160** or selection may be performed manually by a clinician. Signal processing circuitry **160** may use various criteria to automatically select an input, such as, for example, signal quality, signal strength and/or the functionality of the medical devices. Alternatively, clinician may select an input via the input selector switch **113.**

A medical electrode and various medical uses are well know in the art. A medical electrode array is medical device containing a plurality of medical electrodes as described in U.S. Application No. 60/798,842 to Meyer, the contents of which are incorporated herein by reference. Abdominal strain gages, tocodynamometers, intrauterine pressure catheters and ultrasound transducers are also well know in the art.

Lesser known devices include a vacuum pressure sensor, a fetal pulse oximeter, a pH sensor, a cervical dilation sensor, a cervical effacement sensor, a cervical length sensor and a fetal station sensor. A vacuum pressure sensor measures the amount of vacuum applied to a fetal skull by a vacuum extractor, a device used to apply guiding pulls to a fetal scalp during delivery, and the vacuum measured by the vacuum pressure sensor is recorded by an external device. A fetal pulse oximeter measures the oxygen saturation of fetal blood during delivery. A measure of oxygen saturation, in conjunction with the fetal heart rate, can be used to detect abnormalities wherein a clinician may decide to proceed with a cesarean delivery. Similarly, fetal pH, measured with a fetal pH sensor, begins to decrease when oxygen saturation levels decrease. A cervical dilation sensor is used to measure the progress of labor by measuring and recording cervical dilation. A cervical effacement sensor measures the gradual softening or thinning of the cervix during the first stage of labor which may be used to predict the onset of delivery. Similarly, a cervical dilation sensor measures the dilation of the cervix during the first stage of labor. Finally, a fetal station sensor determines the relative positioning between the presenting part of the fetus, whether that be the head, shoulder, buttocks, or feet, and two parts of the maternal pelvis called the ischial spines.

An intrauterine pressure catheter is a common apparatus for measuring the fetal contractions of a maternal abdomen. Various pressure catheter components and systems are described in U.S. Patent No. 5,566,680 to Urion et al., the contents of which are incorporated herein by reference. When using a monitoring device **140** configured to receive a monitoring signal from an IUP catheter it often becomes necessary or desirable to "zero" or "re-zero" the monitoring device **140.** U.S. Patent Application 10/952,942 to Zaiken, titled **Intrauterine Pressure Catheter Interface Cable System and filed on September 29, 2004,** the entire contents of which are incorporated herein by reference, describes use of a pressure catheter, a zero / re-zero apparatus and method of use.

In yet another embodiment of the present disclosure the zero / re-zero function as described in a U.S Utility Patent Application titled **Cable Monitoring Apparatus,** Attorney Docket Number H-KN-00512 (1502-143), concurrently filed on September 29, 2006 with the present application, may be incorporated into a signal replication medical apparatus **100.** Referring to **FIGS. 1** and **2,** zero / re-zero circuitry **130** of the signal replication medical apparatus **100** includes a zero / re-zero selector **132** and zero / re-zero indicator **134.** Clinicians initiate a zero / re-zero of the monitoring device **140** by depressing the zero / re-zero selector **132** on the signal replication medical apparatus **100.** Signal replication medical apparatus **100** replicates a zero-voltage signal on the first output cable **151A** for a predetermined period of time and zero / re-zero indicator **134** indicates that a zero voltage signal is being replicated. Clinicians then depress a zero / re-zero selector on the monitoring device **140** while the zero voltage signal is replicated.

In yet another embodiment of the present disclosure, indicators **I** may correspond to electrodes **121** on the electrode array **120** applied to the maternal abdomen **A.** With reference to **FIG. 1,** indicator circuit **170** is operably connected to the signal processing circuitry **160** and signal processing circuitry **160** may drive the indicators **I** with a signal indicative of at least one operating feature of electrical cable **150.** An operating feature of electrical cable **150** may be associated with the functionality of the electrical cable **150,** the quality of the signal transmitted by electrical cable **150,** or a feature of electrical cable **150** or medical signal.

In yet another embodiment of the present disclosure, one or more of indicators **I** include a light driven by a signal from the signal processing circuitry **160** wherein the signal is indicative of the monitoring signal or the functionality of electrical cable **150.** Indicator **I** may be driven with a signal proportional to the monitoring signal from the medical device, such as an electrode array **120.** Clinicians can troubleshoot problems with an electrical cable **150** or medical device by observing indicator **I** on the signal replication medical apparatus **100.**

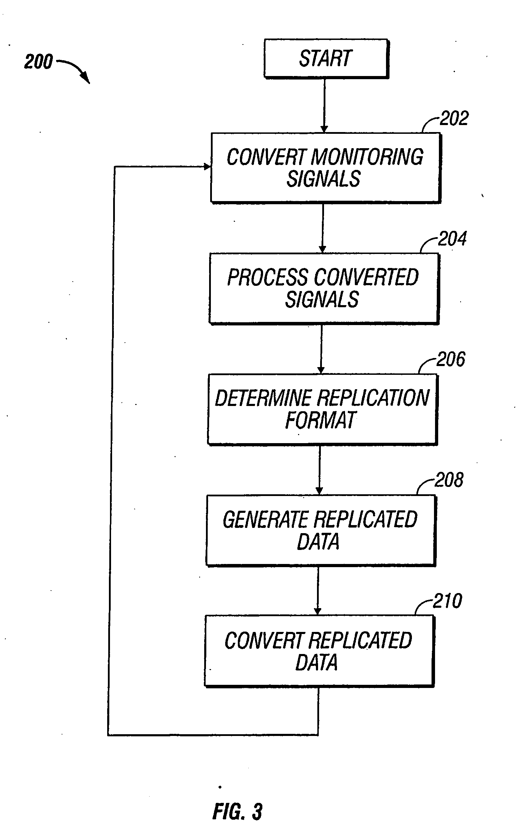

Referring now to **FIG. 3,** programming flowchart **200** illustrates processes executed by the DSP **160B** for performing the functions described herein in accordance with the present disclosure. While the programming flowchart of **FIG. 3** includes multiple embodiments of the present disclosure, the steps executed by the DSP **160B** may be limited to one or more of the various embodiment described herein.

In Step **202** an analog monitoring signal from one or more medical devices is converted from an analog format to a digital representation of the analog monitoring signal. As is known in the art, A/D conversion is not a single step but a real-time process.

In Step **204** the digital representation of the analog monitoring signal is processed. Processing may include extracting a medical signal from a monitoring signal, extracting a medical signal from a plurality of monitoring signals or determining if an extracted medical signal is a valid representation of the expected signal.

In Step **206** the format of replicated data is determined. The extracted medical signal may be replicated as a monitoring signal indicative of a signal having been outputted from a second medical device, wherein the monitoring signal from the second medical device is different that the monitoring signal converted in Step **202.** The replicated data may be a zero-voltage signal, replicated for a predetermined period of time, supplied to the monitoring device **140** to perform a zero / re-zero of the monitoring device **140.** The replicated data may also be indicative of a fault condition recognized by monitoring device **140.**

In Step **208** the replicated data format determined in Step **206** is generated into a digital representation of a monitoring signal. Replicated data may be altered in order to remove an incompatibility between the signal and monitoring device **140,** to increase compatibility of the signal with the monitoring device **140** or to aid a clinician in recognizing an element of the signal.

In Step **210** the replicated data generated in Step **208** is converted into a format recognized by the monitoring device **140.** Format may be analog or digital and may be transmitted to the monitoring device **140** by a cable **151a, 151b** or by any method of wireless transmission used to transmit a signal.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An apparatus for replicating a medical signal, which comprises:
an input adapted to receive at least one medical signal transmitted via a first medical device coupled to the body of a patient;
signal processing circuitry adapted to generate at least one replicated signal from said at least one medical signal, said at least one replicated signal indicative of being transmitted via a second medical device different than the first medical device; and
an output adapted to deliver said at least one replicated signal to an external device configured to receive signals from said second medical device.

2. The medical apparatus according to Claim 1, wherein the signal processing circuitry comprises an ASIC.

3. The medical apparatus according to Claim 1, wherein the signal processing circuitry comprises:
an analog to digital converter for converting said at least one medical signal to a digital format;
a signal processor for processing said at least one medical signal in a digital format wherein data is extracted from said at least one medical signal;
a signal generator for receiving said data extracted by said signal processor and generating therefrom said at least one replicated signal, wherein data relayed by said at least one replicated signal replicates data potentially outputted by said second medical device; and
a digital to analog converter for converting said at least one replicated signal to an analog format.

4. The medical apparatus according to Claim 3, wherein said signal processor is adapted to select one signal of said at least one converted output signal for extracting and processing data relayed by the selected signal.

5. The medical apparatus according to Claim 3, further comprising an electrical connector for outputting the at least one generated signal.

6. The medical apparatus according to Claim 1, wherein the first medical device is selected from a group consisting of at least one medical electrode and a medical electrode sensor array.

7. The medical apparatus according to Claim 6, wherein the second medical device is a tocodynamometer.

8. The medical apparatus according to Claim 6, wherein the second medical device is an intrauterine pressure catheter.

9. The medical apparatus according to Claim 6, wherein the second medical device is an ultrasound transducer.

10. The medical apparatus according to Claim 1, wherein the first and second medical devices are selected from a group consisting of at least one medical electrode, a medical electrode sensor array, an abdominal strain gage, a tocodynamometer, an intrauterine pressure catheter, and an ultrasound transducer.

11. The medical apparatus according to Claim 10,, wherein the group further consisting of a vacuum pressure sensor, a fetal pulse oximeter, a pH sensor, a cervical dilation sensor, a cervical effacement sensor, a cervical length sensor and a fetal station sensor.

12. The medical apparatus according to Claim 1, further comprising one or more indicators wherein said one or more indicators indicate functionality of the first medical device.

13. The medical apparatus according to Claim 12, wherein said one or more indicators are selected from a group consisting of a visual indicator, a auditory indicator and a tactile indicator.

14. The medical apparatus according to Claim 13, wherein said one or more indicators indicates the quality of said at least one medical signal transmitted via said first medical device.

15. The medical apparatus according to Claim 1, further comprising an electrical cable for relaying the at least one signal to the signal processing circuitry.

16. The medical apparatus according to Claim 15, further comprising an electrical receptacle adapted for mating with an end of the electrical cable for performing a diagnostic check on the cable.

17. The medical apparatus according to Claim 1, wherein said at least one output signal includes data selected from a group consisting of heart, fetal, and uterine activity data.

18. The medical apparatus according to Claim 1, wherein the first medical device is a fetal monitoring electrode and the at least one signal is a fetal monitoring signal outputted by said fetal monitoring electrode.

19. The medical apparatus according to Claim 1, wherein the at least one output signal includes a uterine activity signal, and further comprising means for shorting the uterine activity signal to zero.

20. The medical apparatus according to Claim 19, wherein the external device configured to receive signals is a monitor comprising means for performing a zero/re-zero function during a period of time where the uterine activity signal is shorted to zero.

21. The medical apparatus according to Claim 1, further comprising means for powering the medical apparatus.

22. The medical apparatus according to Claim 1, wherein said signal processing circuitry simulates a fault condition wherein said at least one replicated signal is indicative of a signal from said second medical device in a faulted condition.

23. The medical apparatus according to Claim 22, wherein said at least one replicated signal indicative of a signal from said second medical device in a faulted condition induces an alarm in said external device.

24. The medical apparatus according to Claim 1, wherein said signal generation circuitry determines the functionality of said first medical device and replicates a signal in response to said determination.

25. The medical apparatus according to Claim 24, wherein said signal replicated in response to said determination induces an alarm in said external device.

26. The medical apparatus according to Claim 1, wherein at least one alteration of said at least one replicated signal is performed to generate a signal indicative of a signal from said second medical device.

27. The medical apparatus according to Claim 26, wherein said at least one alteration is the inversion of said at least one replicated signal.

28. The medical apparatus according to Claim 26, wherein said at least one alteration is the addition of an offset to said at least one replicated signal.

29. The medical apparatus according to Claim 26, wherein said at least one alteration improves the quality of said at least one replicated signal.

30. The medical apparatus according to Claim 26, wherein said at least one alteration re-scales said at least one replicated signal.

31. The medical apparatus according to Claim 1, wherein said signal generation circuitry receives a plurality of output signals from a first medical device and replicates two signals, indicative of a signals from two separate medical device, wherein said two signals are not the same signal.

32. The medical apparatus according to Claim 1, further comprising:
a second input adapted to receive at least one medical signal transmitted via a second medical device coupled to the body of a patient; and
a means for selecting the source of the replicated signal from said first input or said second input.
